# EUROPEAN PATENT SPECIFICATION

(11) **EP 0 412 621 B1**
(45) Date of publication and mention of the grant of the patent: **03.11.1993**
(21) Application number: 90202160.9
(22) Date of filing: 08.08.1990
(51) Int. Cl.: A01H 4/00

(54) **A method and device for aseptically propagating cells or tissues**
Verfahren und Gerät für die aseptische Vermehrung von Zellen oder Geweben
Procédé et appareil pour la propagation aseptique de cellules ou tissus

(30) Priority: 08.08.1989 NL 8902032
(43) Date of publication of application: 13.02.1991
(73) Proprietor: Jethar Deelnemingen B.V., NL-1426 AJ De Hoef (NL)
(72) Inventor: van Oeveren, Jacobus Johannes, B-4890 Malmédy (BE)
(74) Representative: Boelsma, Gerben Harm, Ir.

(56) References cited:
- EP-A- 0 232 628
- EP-A- 0 282 230
- EP-A- 0 316 777
- GB-A- 852 114
- US-A- 4 215 513

## Description

The invention relates to a method for aseptically propagating cells and tissue, more particular for the vegetative propagation of plantlets, whereby in a sterile environment starting material is placed in individual membrane capsules, into which an amount of nutrient medium for the material to be propagated or to be rooted in advance, upon which the capsules are sealed.

Such a method is disclosed in EP-A-0 282 230. Working with permeable capsules in which plantlets, obtained by division, are allowed to propagate and root respectively in a separate individual space, has brought significant progress in growth as compared with the in-vitro method which is still applied on a large scale and with which large numbers of plantlets to be propagated and rooted respectively, are "placed to culture" in containers formed of a suitable plastics material. A favourable aspect with the "capsule-culture" is to be seen in that infections with the individual plantlets cannot spread to adjacent plantlets, as is the case in larger containers.

In addition to working with individual capsules it has also been known to use multiple capsules (see EP-A-0 316 777 and US-A-4 215 513). Such multiple capsules or capsule chains may be formed by folding a continuous web of a suitable membrane material along its central longitudinal axis and then providing a plurality of seals, which extend mutually parallel and perpendicular to the folding line and divide the folded web into a number of juxtaposed capsule cells. After (longitudinal) sealing such multiple capsules are usually stored, in an upright or suspended position, in a culture room, the latter being conditioned e.g. by lamps. The improved propagating technics with capsules, however, has not lead to changes in the circumstance, that substantially all of the successive steps of the method have to be performed by hand.

The invention aims at a further development of the "capsule method" in the sense that the actions to be performed by hand may be reduced to a minimum and more particular to those actions, which are related to the production - by cutting - of the starting material for the desired propagation and rooting respectively.

According to the present invention this aim is achieved with a method having the features mentioned in the characterising clause of the main claim.

With the exception of the step of "presenting" the starting material, the actions involved with this method may be all realised in an effective manner with mechanical means.

The method of the present invention can be considered as the basis for a continuous production system for the vegetative propagation of plants according to the so called tissue culturing principle. The human interference in a production line to be set up according to the present method is limited to the "presenting" of the plantlets to be propagated, whereby the "presentator" may also maintain the required human watch on the system.

It is essential with the method according to the invention, that the successive actions are carried out in a sterile atmosphere. With the method of the invention this requirement may be simply satisfied by positioning the successive stations between an upper and a lower confinement extending in the production line direction, between which a sterile air flow is maintained.

The invention also relates to a device for performing the above method.

The device according to the present invention is characterized by the features mentioned in claim 3.

The invention will be hereinafter further described by way of example with reference to the drawings.

The invention will be hereinafter further described by way of example with reference to the drawing.
Fig. 1 is a diagramatic plan view of a first section of the device for carrying out the method of the invention, in which the steps of opening the capsules, filling the same with nutrient medium, inserting the plantlets to be propagated or to be rooted, and the sealing of the capsule chain are successively carried out;
fig. 2 is a diagramatic horizontal cross-section of a detail of the device of fig. 1;
fig. 3 is a diagramatic plan view of the last section of the device, in which the filled and sealed capsule chain is stored in a container;
fig. 4 is a side view of the device of fig. 1;
fig. 5A and 5B represent a cross-sectional view along the line V - V in fig. 1 and a side view on an enlarged scale of that section of the device, in which the filling with nutrient medium is taking place;
fig. 6A and 6B represent a cross-sectional view along the line VI - VI in fig. 2 and a side view on an enlarged scale of that section of the device, in which the plantlets to be propagated or to be rooted are transferred by manipulators from an offset handling spot towards the capsules of the capsule chain; and
fig. 7A and 7B represent a side view and a front view respectively of a carrying beam with manipulators in a modified embodiment.

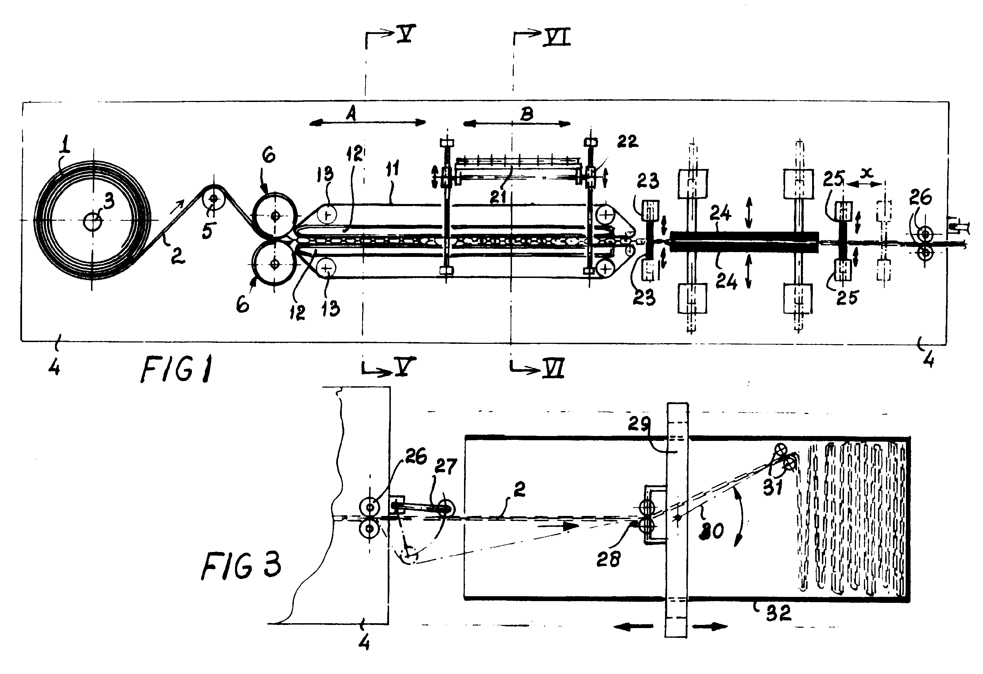

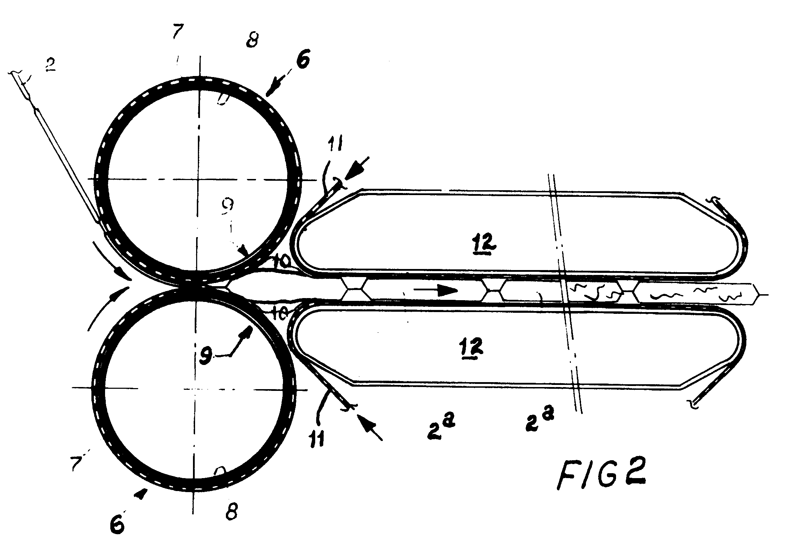

In reality the sections of the device shown in fig. 1 and 3 are connected to one another along a straight production line. A number of devices may be positioned in parallel to one another, one device e.g. forming a production line for plantlets to be propagated and a second one serving as a production line for plantlets to be rooted. These production lines may be positioned longitudinally offset through the length of a treatment station, while their drive means may be coupled.

In the drawing 1 designates a capsule chain 2 which has been wound to a supply roll. The capsule chain 2 consists of a double walled membrane foil of plastics material, which is divided into a large number of juxtaposed compartments (the "capsules") which are separated by individual seals extending perpendicular to the longitudinal direction of the foil. Such a capsule chain may e.g. be realised by starting from an extruded tube, which is cut, in flat folded condition, into two halves along its longitudinal axis.

The initial width of the foil or the flat-folded tube respectively is selected so that the height and the width of the capsules will be of a size which may vary between 100 and 200 mm and 30 and 60 mm respectively, whereas the width of the seals between the capsules is about 4 mm. The membrane thickness to be used may roughly vary between 20 and 30 .

The supply roll 1 is mounted onto a base 4 for rotation about a vertical axis 3.

The capsule chain 2 is guided in an upright position via a guide roller 5 towards the inlet area between two cooperating drawing rollers 6. In the embodiment shown the drawing rollers 6 each comprise an outer drum 7 provided with perforations, which is mounted for rotation about a vertical axis around a fixed drum 8, which is connected to a vacuum conduit not shown. The perforations of the outer drum are, for the greater part of the circumference of the drum, interiorly covered by the fixed drum. Only in the outlet area indicated at 9 in fig. 2, the perforations of the outer drum are in free communication with the interior of the fixed drum, so that a vacuum, created within the latter interior space may act upon the emerging capsule chain to cause the capsule, which is momentarily within the outlet area, to open.

The drawing rollers 6 are driven in the arrow direction by drive means not further shown.

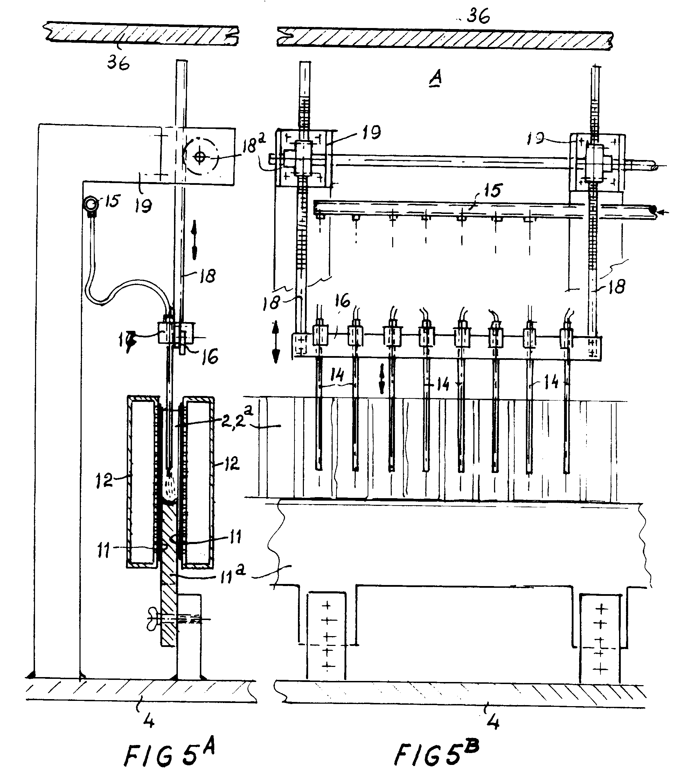

As seen from the outlet area 10 the capsule chain 2 with its opened capsules 2a is guided into the gangway between a two cooperating, endless conveyor belts 11. The parallel opposite runs of these conveyor belts are guided along the opposite, perforated walls of two vacuum chambers 12, positioned on both sides of the path of the capsule chain. The belts 11 are guided around the ends of the vacuum chambers 12 and are further passed over guide rollers 13, tensioning- and drive rollers, which are all rotatably supported on the base 4. The drive means for the belts may be accomodated under the base 4. The chambers 12 are connected to a vacuum source not further shown. The underpressure created within the chamber 12 causes the walls of the opened capsule chain to be sucked against the cooperating runs of the conveyor belts 11. The capsule chain 2 will thus be frictionally engaged by the conveyor belt 11 and taken along in the conveying direction. The lower edge of the capsule chain 2, which forms the common bottom of the capsules 2a, is supported on a vertically adjustable support profile 11a (vide fig. 5).

Over the first part A of the path of the capsule chain 2 confined by the conveyor belt 11, there is provided a battery of e.g. eight vertical filling pipes 14, which are positioned side by side as seen in the travelling direction (vide fig. 5). These filling pipes 14, which are mutually spaced in correspondence with the pitch of the capsules 2a, are e.g. formed by flexible tubes, which are connected to a common, pressurized supply vessel 15 (vide fig. 5), so as to be supplied, under pressure, with a liquid nutrient medium. The filling tubes are mounted on a carriage 16, which is adapted to be moved vertically up and down by means of toothed racks 18 and pinions 18a, accomodated in fixed guides 19. Each of the filling tubes is attached to a rider-like mounting element 17. The mounting elements may be mutually spaced at the desired pitch and fixed on the carriage 16. Furthermore the filling tubes 14 are each provided with an e.g. solenoid operated tube clamp (not shown). These tube clamps are arranged in a control circuit designed to - during a standstill of the capsule chain - cause the clamps to open for a predetermined and adjustable period of time so as to permit the desired quantity of liquid nutrient medium to flow out of the associated filling tubes 14 into the capsules 2a thereunder. The carriage 16 is also taken up in a control circuit adapted to cause the carriage with the battery of filling tubes 14 to lower into the underlying capsules 2a immediately after the capsule chain 2 has come to a standstill, and to re-elevate the same after filling.

The drawing rollers 6 and the part of the conceyor belts 11 extending along the trajectory A with the battery of filling tubes 14 thereover can be considered as that section of the device which is designated in the claims with the term "first station".

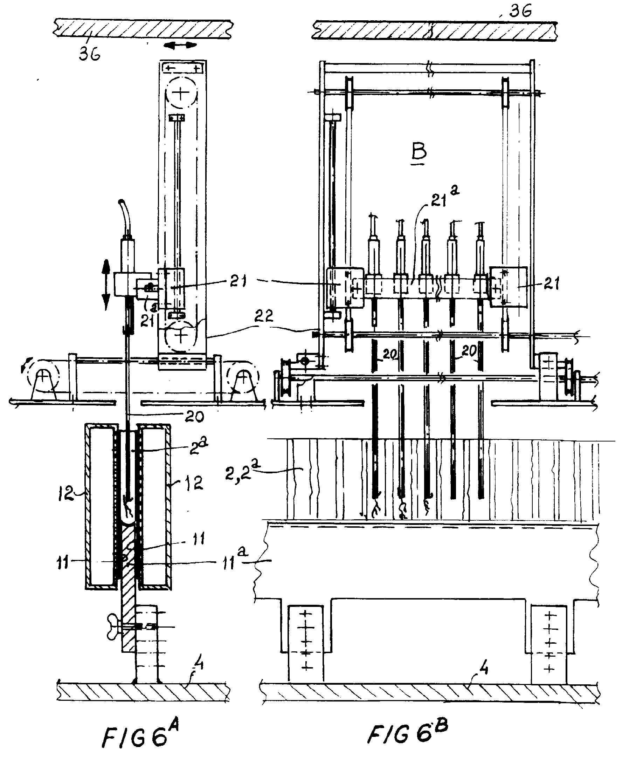

Over the trajectory indicated at B there is a battery of (in this case) eight manipulators 20. These manipulators are mounted side by side on a support 21 with a mutual spacing which corresponds to the pitch of the opened capsules, said support being mounted for movement up and down along a carriage 22. In turn the carriage 22 is - with the manipulators 20 in the upper positions - mounted to move back and forth in a horizontal direction transverse to the travelling direction of the capsule chain (vide fig. 1 and 6) between a position in which the manipulators 20 are positioned right over a series of eight opened capsules containing a quantity of nutrient medium, and a position, in which the manipulators are in a position laterally offset with respect to the travelling path of the capsule chain over a location, where a plantlet to be propagated, or to be rooted respectively is presented by hand to each of the manipulators.

The manipulators 20, the support 21 and the carriage 22 are parts of a plantlet-inserting automate, which comprises various drives. These drives and the travelling mechanism for the capsule chain are set in such a (mutual) timed relationship, that the period, in which the capsule chain is making a displacement step covering the joint width of eight capsules in the travelling direction, is used for having a plantlet gripped by each of the manipulators, causing the manipulators to move upwardly and then causing the carriage 22 to move transversely until over the capsule chain.

In a simple embodiment, which is suitable for certain types of plantlets, the manipulators are formed by tubes which have their upper ends connected to a vacuum source in a flexible manner (fig. 1,4 and 6). The connection to the vacuum source is switched on as soon as the support 21 with the vacuum tube 20 has arrived in a position over the handling spot, where the plantlets to be inserted are "presented". The underpressure within the tube causes the presented plantlets to be sucked against the lower ends of the tube. On the moment the capsule chain is coming to a standstill, the tubes 20 may be lowered into the opened and filled capsules. At the end of the lowering movement the connection with the vacuum source is interrupted, so that the transferred plantlets are released and will be left in their breeding ground when the tubes 20 are moving upwardly again.

The trajectory B mentioned herein above corresponds to the "second station" mentioned in the claims. Due to mounting the manipulators 20 on a supporting beam 21a which is removably attached to the support 21, it is possible to take a loose supporting beam 21a with manipulators 20 on the handling spot and load the same with plantlets to be transferred later, while another supporting beam 21a on the support 21 is carrying out the step of introducing an earlier series of plantlets into the capsule chain. The empty supporting beam 21a returning to the handling spot after insertion of the plantlets may be exchanged for a "loaded" one in order to be reloaded while the plantlet inserting automate may start the next inserting step. In this way gaps in the productivity on the handling spot are avoided. Furthermore, the manipulators or tubes 20 respectively may be individually removably mounted to the supporting beam 21a.

The trajectory B, described herein above, may extend between a separate set of conveyor belts with associated vacuum chambers, the inlet area of which conveyor belts being positioned immediately adjacent the outlet area of the conveyor belts in trajectory A. These separate conveyor belts in trajectory B may cover a smaller height.

The capsule chain provided with plantlets in trajectory B will be displaced, in the next travelling step, with its upper longitudinal edge portions in a heat sealing station, which is called "third station" in the appending claims. In this sealing station the capsule chain is supported by the supporting profile 11a and on both sides of the longitudinal edge portions there are a first pair of clamping jaws 23, a pair of sealing beams 24 and a second pair of clamping jaws 25. The first pair of clamping jaws 23 is taking a fixed longitudinal position adjacent the outlet area of the trajectory B, whereas the sealing beams 24 are also taking a fixed longitudinal position. The cooperating jaws of said first pair of clamping jaws 23 are movable one towards the other transversely so as to clamp the capsule chain along the upper longitudinal edges. In a similar manner the cooperating sealing beams 24 are movable towards one another in a transverse direction so as to seal the two walls of the capsule chain adjacent the upper edge together.

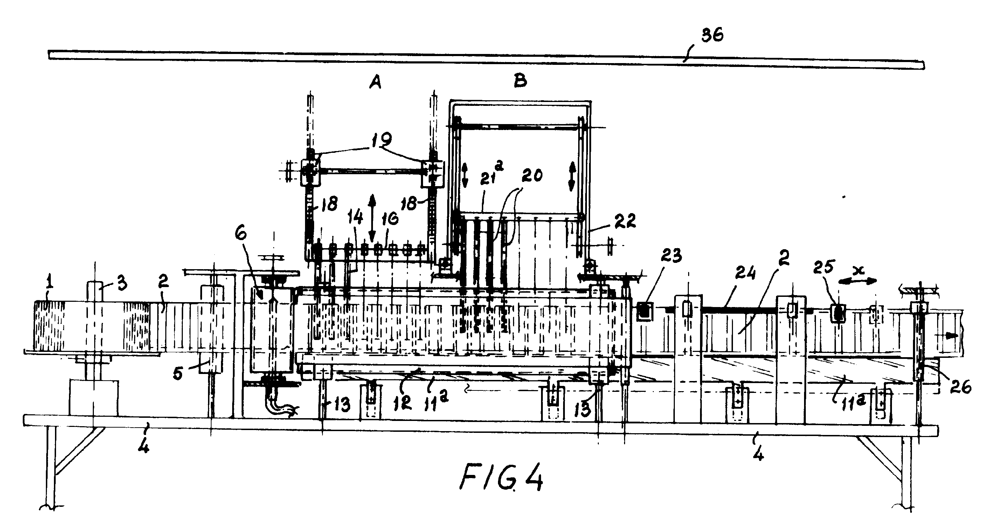

The second pair of clamping jaws 25 is not only movable in a transverse direction to clamp the capsule chain 2 along its upper edge, but may also be displaced through a distance x (fig. 1 and 4) in the travelling direction, so as to stretch the clamped capsule chain and bring the same in a condition, in which the sealing beams 24 may then produce an excellent seal adjacent the upper edge of the capsule chain.

The actuation of the various clamping jaws and sealing beams is effected in such a way that - while the conveyor belts are stationary - the cooperating jaws of the clamping jaw pairs 23 and 25 are moved together first to clamp the stationary capsule chain, thereafter the second pair of clamping jaws 25 is displaced in the travelling direction to stretch the capsule chain 2, and thereafter the cooperating beams of the sealing beam pair 24 are brought together so as to produce the desired longitudinal seal. It will be clear that the length of the sealing beams has to be selected such that it will be able to cover at least eight capsules in the stretched condition of the capsule chain; preferably there is a certain excess in length, so that the seals produced in successive steps will slightly overlap one another.

The sealed capsule chain 2 is pulled through the sealing stations by means of two continuously driven drawing rollers 26. The rollers 26 are connected to a drive shaft (not further shown) by means of a slip coupling adjusted to a small torque, so that they are permitted to remain stationary when the conveyor belts 11 are at a standstill. From the drawing rollers 26 the capsule chain is passed via a laterally flexible guide roller 27 between two cooperating slipping drive rollers 28 positioned in alignment with the heat sealing station, from where the capsule chain 2 is engaged by a gripping arm 30 provided with guide rollers 31 and swingably mounted on a carriage 29 to be folded to a zig-zag package which is deposited in a storage container 32. The storage container 32 may be moved in the man travelling direction underneath the fixed carriage 29 at a speed or in steps which correspond with the degree of loading of the container.

With reference to the device described herein before, the part extending up to the outlet of the heat sealing station is installed in a so-called "sterile cloud", which is created as a vertical sterile air flow between a panel 36 covering the device on the upper side, and the platform 4, which serves as a supporting base for the various parts of the device. The means required for this purpose are not further shown in the drawing and may be accommodated in the space above the panel 36.

The "sterile cloud" also extends up to the handling spot, where the plantlets to be transferred are presented by hand to the Manipulators.

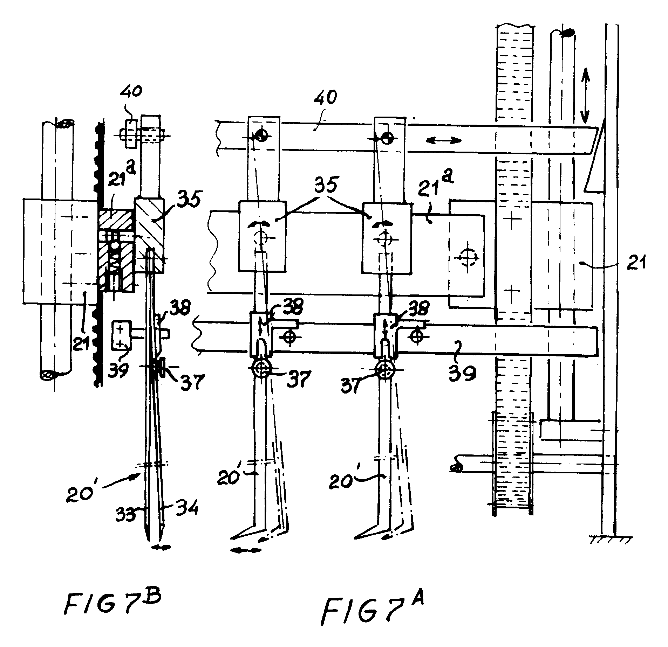

Fig. 7A and 7B show an alternative of the vacuum tubes shown in fig. 6A and 6B and serving as manipulator. In the modified embodiment use is made of tweezer-like manipulators 20', the tweezers 33 and 34 of which are fastened in a mounting block 35, which is removably and pivotally "clicked" on the supporting beam 21a. On the handling spot the tweezers 20' are individually taken from the empty supporting beam 21a, so as to be "loaded" with a plantlet to be transferred and then be put back on its place onto the supporting beam 21a preparing for the next transfer. Advantageously use can be made of a pair of tweezers of the type, of which he tweezers of spring-urged in an open position. On the handling spot the pair of tweezers can be simply closed by the "presentator" by pinching the tweezers together and then locked in the closed position e.g. by operating a locking slide 38 cooperating with the locking pin 37. A pair of tweezers of this type is known per se for manual use.

A battery of tweezers loaded with plantlets may be lowered in a similar manner as described with reference to fig. 6A and 6B, into the individual capsules during a standstill of the capsule chain.

In order to release the transferrd plantlets in their growing grounds, the tweezers have to be opened first and then be swung laterally to become completely free from the released plantlet and be permitted to be pulled upwardly again.

The opening of the tweezers is effected by means of a sliding bar 39 fastened to the support 21 in the path of the locking slides, said sliding bar being vertically movable through a slide distance relative to the supporting beam 21a. If desired, the sliding bar 39 could form a fixed connection between the parts of the support 21, in which case the vertical movement required for the unlocking could be obtained by blocking the supporting beam 21a in the final stage of the lowering movement, while the support 21 is continuing through a slide distance towards its lower end position. The swinging movement required for releasing the plantlets is effected by an actuation rod 40, which is mounted, preferably on the supporting beam 21a, for movement in the arrow direction. It will be clear, that the unlocking- and swinging means have to be actuated in the correct order. This requirement may be easily satisfied when use is made of electromagnets as drive means.

## Claims

1. A method for aseptically propagating cells and tissue, more particular for the vegetative propagation of plantlets, whereby in a sterile environment starting material is placed in individual membrane capsules (2a), into which an amount of nutrient medium for the material to be propagated or to be rooted has been introduced in advance, upon which the capsules (2a) are sealed, characterized in that a continuous capsule chain (2) is caused to gradually unwind from a flat folded storage condition and to move in a substantially upright position along a number of stations, viz. a first station (A), where the capsule chain (2) passes through the space between two upright walls (12), while an underpressure is created within the latter space, which underpressure causes the opposing membrane walls of the capsule chain (2) to spread apart, after which the thus opened capsule cells (2a) are filled with a quantity of nutrient medium with the aid of filling means (14-18) which are introduced from above; a second station (B), where a plantlet to be propagated or rooted respectively is placed from above in each of the capsule cells (2a) which are previously provided with nutrient medium; a third station where the capsule cells (2a) containing culture material are closed by a horizontal seal and a fourth station where the filled and closed capsule chain (2) is placed in a storage container (32) in a horizontally packed condition.

2. A method according to claim 1, characterized in that the capsule chain (2) is moved through a surrounding sterile cloud on the trajectory comprising the capsule chain (2) in the flat folded condition, and at least the first three stations (A, B, 24).

3. A device for carrying out the method according to claims 1 - 2, characterized by a base (4) for a cylindrically shaped capsule chain (2) supply, adapted for unwinding about a vertical axis (3), with guide means (6) for guiding said chain (2) towards an inlet slit of a gangway confined between two vertical walls (12), means being provided for creating an underpressure within said gangway via openings provided in said walls, means (14-18) for supplying a liquid fluid being provided at a first location (A) over said gangway, the latter means (14-18) being adapted to enter downwardly into the capsule cells (2a) to be opened by said underpressure, means (20; 20') being provided (B) in a second location over said gangway and adapted to grip a plantlet to be propagated and adapted to be lowered into said capsule cells (2a) and to be elevated upon release of the plantlet, a pair of sealing beams (24) being located at the outlet of said gangway, along the upper side of the path of the capsule chain (2), and one or more gripping arms (30) or guide arms being mounted at the outlet of said beams (24), adapted to engage the capsule chain after sealing and wind or fold the same to a compact pack.

4. A device according to claim 3, characterized in that said base (4) extends in the longitudinal direction of the device up to the outlet area of the heat sealing station (24), a panel (36) being provided at a distance over said base (4) with means provided there above for creating a vertical air flow between said panel and said base (4) which surrounds the device.

5. A device according to claims 3 - 4, characterized in that the capsule chain (2) is supported with its bottom bearing on a support profile (11a) positioned under the gangway.

6. A device according to claim 5, characterized in that the support profile (11a) is extending beyond the heat sealing station (24).

7. A device according to claims 3 - 4, characterized in that the filling means (14-18) are formed by filling tubes (14), which are connected, by means of flexible tubes, to a supply reservoir (15) which may be adjusted to a predetermined filling pressure, each flexible tube being provided with a solenoid operated tube clamp.

8. A device according to claims 3 - 7, characterized in that the means for gripping a plantlet to be transferred, are formed by pipes (20) connectable to a vacuum source.

9. A device according to claim 8, characterized in that the pipes (20) are removably suspended to a supporting beam (21a), which in turn is removably mounted to a support which is fastened to a vertically movable carriage (21), said carriage being also movable in a horizontal direction transverse to the travelling direction of the capsule chain (2) between a position over the capsule chain and a laterally offset position over a handling spot.

10. A device according to claims 3 - 6, characterized in that the means for engaging and transferring the plantlets to be propagated or to be rooted respectively are formed by tweezer-like (hand-actuated) manipulators (20'), said manipulators being removably suspended to a horizontal supporting beam (21a) and mounted thereon to swing through a small angle from their vertical position within the plane through the travelling direction of the capsule chain (2), said supporting beam (21a) being in turn removably mounted to a support which is fastened to a vertically movable carrier (21), said carrier (21) being also movable in a direction transverse to the travelling direction of the capsule chain (2) between a position over the capsule chain and an offset position over a handling spot, means (39) being provided to release the tweezers (33, 34) from the gripping position.

11. A device according to claim 10, whereby the tweezers (33, 34) may be manually locked in the closed position by means of a locking slide (38) cooperating with a locking pin (37), characterized in that the means for releasing the tweezers from the gripping position are formed by a sliding rod (39), which is vertically movable relative to the supporting beam (21a) so as to jointly control the locking slides (38) of the tweezers (33, 34).

## Patentansprüche

1. Verfahren zur aseptischen Vermehrung von Zellen und Gewebe, insbesondere zur vegetativen Vermehrung von Pflanzen, wobei man in einer sterilen Umgebung Ausgangsnaterial in gesonderte Membrankapseln (2a) einbringt, in denen vorab eine Menge Nahrungsmittel für das zu vermehrende oder bewurzelnde Material eigebracht worden ist, worauf die Kapseln (2a) zugeschweist werden, dadurch gekennzeichnet, dass man eine kombinierliche Kapselkette (2) sich allmählich aus einem flach-gefalteten Lagerungszustand abwickeln lässt und in etwa senkrechter Lage an einer Anzahl von Stationen entlang führen lässt und zwar einer ersten Station (A), in der die Kapselkette (2) durch den Zwischenraum zwischen zwei senkrechten Wänden (12) hindurchbewegt, während in diesem Zwischenraum ein Unterdruck erzeugt wird, der das Auseinandergehen der einandergegenüber stehenden Membranwände der Kapselkette (2) bewirkt, worauf die so geöffnete Kapselzellen (2a) mittels von oben her einführbarer Füllmitteln (14-18) mit einer Menge Nahrungsmittel ausgefüllt werden können; einer zweiten Station (B), in der man je ein zu vermehrenden oder zu bewurzelnden Pflanzchen von oben her hintereinander in jede mit einem Nahrungsmittel versehene Kapselzelle (2a) hineinbringt; einer dritten Station, in der die mit Zuchtmaterial versehenen Kapselzellen (2a) mit einem waagerechten Schweissnaht geschlossen werden; und einer vierten Station in der die gefüllte und geschlossene Kapselkette (2) in einem waagerecht zusammengepacktem Zustand in einen Behälter aufgenommen wird.

2. Verfahren nach anspruch 1, dadurch gekennzeichnet, dass die Kapselkette (2) im Bereich des flach-gewickeltem Zustandes desselben und mindestens der ersten drei Bearbeitungsstationen (A, B, 24) durch eine die Kapselkette (2) umhüllende sterile Walke hindurch bewegt.

3. Vorrichtung zur Durchführung des Verfahrens nach Ansprüchen 1-2, gekennzeichnet durch eine Fussplatte (4) für einen zylindrisch geformten Kapselkettenvorrat (2), der um eine senkrechte Achse (3) abgewickelt werden kann, mit Führungsmitteln (6) zum Führen der Kette (2) zu einem Eintrittsschlitz eines zwischen zwei senkrechten Wänden (12) begrenzten Durchgangsraums für das sich abwickelnde Kapselband, wobei Mittel zum Erzeugen - über Löcher in jenen Wänden - eines Unterdrucks im Durchgangsgang vorhanden sind, wobei an einer ersten Stelle (A) oberhalb des Durchgangsraumes Mittel (14-18) zum Zuführen einer Flüssigkeit vorhanden sind, welche Mittel (14-18) von oben her in die Flüssigkeit des Unterdrucks zu öffnenden Kapselzellen (2a) hineintreten können; in einer zweiten Stelle (B) oberhalb des Durchgangsraumes Mittel (20; 20') vorhanden sind, die ein zu vermehrendes Pflanzchen greifen können, in die Kapselzellen (2a) gesenkt werden können und nach Freigeben des Pflanzchen wieder hochgezogen werden können, und wobei an der Austrittsseite des Durchgangsraums - längs der oberen Seite der Durchgangsstrecke der Kapselkette (2) - ein Satz von Schweissbalken (24) vorgesehen ist an dessen Ausgangsseite ein oder mahrere Greifarme (30) oder Führungsarme vorhanden sind, welche die Kapselkette nach dem Zuschweissen derselben anfassen können und zu einem kompakten Paket aufwickeln bezw. zusammenfalten können.

4. Vorrichtung nach Anspruch 3, dadurch gekennzeichnet, dass sich die Fussplatte (4) in der Längsrichtung der Vorrichtung bis zum Bereich der Schweisstation (24) erstreckt, wobei im abstand oberhalb der Fussplatte (4) eine Platte vorgesehen ist, mit über derselben vorgesehenen Mitteln zum Erzeugen eines die Vorrichtung umgebenden senkrechten Luftstroms zwischen jener Platte und der Fussplatte (4).

5. Vorrichtung nach Ansprüchen 3-4, dadurch gekennzeichnet, dass die Kapselkette (2) mit seiner Bodenteil auf einem unter dem Durchgangsraum angeordneten Stützprofil (11a) aufliegt.

6. Vorrichtung nach Anspruch 5, dadurch gekennzeichnet, dass sich das Stützprofil (11a) bis ausserhalb der Schweisstation (24) erstreckt.

7. Vorrichtung nach Ansprüchen 3-4, dadurch gekennzeichnet, dass die Füllmittel (14-18) durch Füllrohre (14) gebildet sind, welche mittels biegsamer Schläuche an einem auf einen vorbestimmten Fülldruck einstellbaren Vorratbehälter angeschlossen sind, wobei jeder Schlauch mit einer elektromagnetisch bedienbaren Verschlussklemme versehen ist.

8. Vorrichtung nach Ansprüchen 3-7, dadurch gekennzeichnet, dass die Mittel zum Greifen eines zu übertragenden Pflanzchen durch an einer Vakuumquelle anschliessbare Rohre (20) gebildet werden.

9. Vorrichtung nach Anspruch 8, dadurch gekennzeichnet, dass die Rohre (20) abnehmbar auf einem Stützbalken (21a) aufgehängt sind, wobei letzterer abnehmbar auf einer an einem senkrecht bewegbaren Wagen (21) befestigten Stütze montiert ist, welcher Wagen ebenfalls in waagerechter Richtung quer zur Fortbewegungsrichtung der Kapselkette (2) zwischen einer Lage senkrecht über der Kapselkette und einer seitlich versetzten Lage oberhalb einer Handwerkstelle bewegbar ist.

10. Vorrichtung nach Ansprüchen 3-6, dadurch gekennzeichnet, dass die Mittel zum Aufnehmen und Übertragen der zu vermehrenden bezw. zu bewurzelnden Pflanzchen durch pinzettenartige (mit Hand bedienbare) Manipulator (20') gebildet werden, welche Manipulator abnehmbar auf einem waagerechten Stützbalken (21a) aufgehängt sind, in der Weise, dass sie um einen kleinen Winkel aus ihrer senkrechten Lage innerhalb der Ebene durch die Fortbewegungsrichtung der Kapselkette (2) schwenken können, welcher Stützbalken (21a) an sich abnehmbar auf einer Stütze angeordnet ist, welche auf einem senkrecht bewegbaren Wagen (21) befestigt ist, welcher Wagen (21) ebenfalls in einer Richtung quer zur Fortbewegungsrichtung der Kapselkette (2) zwischen einer Lage senkrecht über der Kapselkette und einer versetzen Lage oberhalb einer Handwerkstelle bewegbar ist, wobei Mittel (39) vorhanden sind um die Pinzetten (33, 34) aus der Greiflage freizugeben.

11. Vorrichtung nach Anspruch 10, wobei die Pinzetten (33, 34) mittels eines mit einem Sperrbolzen (37) zusammenarbeitenden Sperrschiebers (38) mit Hand in der Schliesslage sperrbar ist, dadurch gekennzeichnet, dass die Mittel zum Freigeben der Pinzetten aus der Greiflage durch eine Schubstange (39) gebildet werden, welche senkrecht mit bezug auf den Stützbalken (21a) bewegbar ist zur Bedeinung sämtlicher Sperrschieben (38) der Pinzetten (33, 34).

## Revendications

1. Procédé de multiplication aseptique de cellules et de tissus, plus particulièrement pour la manipulation végétative de plantules, qui consiste à placer dans un environnement stérile de la matière de départ dans des capsules de membranes (2a), dans lesquelles une certaine quantité de milieu nitritif pour la matière à multiplier ou à enraciner a été introduite à l'avance, puis à sceller les capsules (2a), caractérisé en ce qu'il consiste à faire se dérouler peu à peu une chaîne (chapelet) (2) continue de capsules d'un état de stockage plié à plat et à les faire se déplacer dans une position sensiblement verticale le long d'un certain nombre de postes, à savoir un premier poste (A) où la chaîne (2) passe dans l'espace compris entre deux parois (12) verticales, une dépression étant créée dans ce dernier espace, cette dépression provoquant l'ecartment des parois opposées des membranes de la chaîne (2) de capsule, puis les cellules (2a) de capsules ainsi ouvertes sont emplies d'une certaine quantité de milieu nutritif à l'aide de moyens de remplissage (14 à 18), qui sont introduits par le haut; un deuxième poste (B) dans lequel un plantule à multiplier au à enraciner respectivement est placé par le dessus dans chacune des cellules (2a) de capsules qui sont munies préalablement de milieu nutritif; un troisième poste dans lequel les cellules (2a) de capsules contenant de la matière de culture sont fermées par un joint horizontal et un quatrième dans lequel la chaîne (2) de capsules emplie et fermée est placée dans un récipient (32) de stockage, à l'état tassé honzontalement.

2. Procédé suivant la revendication 1, caractérisé en ce que la chaîne (2) de capsules est déplacée dans un nuage stérile sur la trajectoire comprenant la chaîne (2) de capsules à l'état plié à plat et au moins les trois premiers postes (A, B, 24).

3. Dispositif pour la mise en oeuvre du procédé suivant les revendications 1 au 2, caractérisé par une base (4) pour une alimentation d'une chaîne (2) de capsules de forme cylindrique, agencée de manière à se dérouler d'un axe (3) vertical avec des moyens de guidage (6) destinés à guider la chaîne (2) vers une pente interne d'un passage confiné entre deux parois (12) verticales, des moyens étant prévus pour créer une dépression dans ce passage par des ouvertures ménagées dans les parois, des moyens (14-18) d'alimentation en fluide liquide étant prévus en un premier emplacement (A) sur ce passage, des derniers moyens (14-18) étant destinés à pénétrer vers le bas dans les cellules (2a) de capsules destinées à s'ouvrir sous la dépression, des moyens (20; 20') étant prévus (B) en un second emplacement dans ce passage et étant agencés pour appréhender une plantule à multiplier et étant adaptés de manière à être à être abaissés dans les cellules (2a) de capsule, et à être soulevés après avoir relâché la plantule, duex barres de scellement (24) étant disposées à la sortie du passage, le long du côté supérieur du trajet de la chaîne (2) de capsules et un au plusieurs bras (3) de préhension au bras de guidage étant montés à la sortie des barres (24), de manière à venir en contact avec la chaîne de capsules après scellement et enroulement ou pliage de celle-ci en un emballage compact.

4. Dispositif suivant la revendication 3, caractérisé en ce que la base (4) s'étend dans la direction longitudinale du dispositif jusqu'à la sortie du poste (24) de scellement à chaud, un panneau (36) étant prévu à une certaine distance au-dessus de la base (4) et étant muni de moyens pour créer un courant d'air vertical entre le panneau et la base (4) qui entoure le dispositif.

5. Dispositif suivant la revendication 3 au 4, caractérisé en ce que la chaîne (2) de capsules est supportée par son fond portant sur un profilé support (11a) disposé endessous du passage.

6. Dispositif suivant la revendication 5, caractérisé en ce que le profilé support (11a) s'étend audelà du poste (24) de scellement à chaud.

7. Dispositif suivant la revendication 3 ou 4, caractérisé en ce que les moyens de remplissage (14-18) sont formés de tubes de remplissage (14) qui communiquent au moyen de tubes souples avec un reservoir (15) d'alimentation qui peut être réglé à une pression d'alimentation déterminée à l'avance, chaque tube souple étant muni d'une pince de serrage de tube fonctionnant à l'aide d'un électro-aimant.

8. Dispositif suivant l'une des revendications 3 à 7, caractérisé en ce que les moyens de préhension de plantules à transférer sont formés de conduits (20) pouvant communiquer avec une source de vide.

9. Dispositif suivant la revendication 8, caractérisé en ce que les conduits (20) sont suspendus de manière amovible à une barre (21) support qui est montée, à son tour, de manière amovible sur un support, lequel est fixé à un chariot (21) mobile verticalement, ce chariot étant également mobile dans sa direction horizontale transversale à la direction de déplacement de la chaîne (2) de capsules, entre une position au-dessus de la chaîne de capsules et une position décalée latéralement au-dessus d'un point de manipulation.

10. Dispositif suivant l'une des revendications 3 à 6, caractérisé en ce que les moyens destinés à venir en contact avec les plantules à multiplier au à enraciner et à les transférer sont formés, respectivement, de manipulateurs (20') analogues à des pincettes (actionnées à la main), ces manipulateurs étant suspendus de manière amovible à une barre (21a) horizontale de support et montés sur celle-ci de manière à basculer sur un petit angle depuis leur position verticale dans le plan dans la direction de passage de la chaîne (2) de capsule, la barre de support (21a) étant montée, à son tour, amovible sur un support qui est fixé à un chariot (21) mobile verticalement, ce chariot étant également mobile dans une direction transversale à la direction de déplacement de la chaîne (2) de capsules entre une position au-dessus de la chaîne de capsules et une position decalée au-dessus d'un point de manipulation, des moyens (39) étant prévus pour relâcher les pincettes (33, 34) de la position de préhension.

11. Dispositif suivant la revendication 10, dans lequel les pincettes (33, 34) peuvent être verrouillées manuellement dans la position fermée au moyen d'un coulisseau (38) de verrouillage coopérant avec une broche (37) de verrouillage, caractérisé en ce que les moyens pour relâcher les pincettes de la position de préhension sont formés par une tige coulissante (39) qui est mobile verticalement, par rapport à la barre de support (21a), de manière à commander conjointement les coulisseaux (38) de verrouillage des pincettes (33, 34).
